# EUROPEAN PATENT APPLICATION

(11) **EP 1 941 828 A1**
(43) Date of publication of application: **09.07.2008**
(21) Application number: 06811433.9
(22) Date of filing: 06.10.2006
(51) Int. Cl.: A61B 1/00, A61B 5/07

(54) **EXAMINEE INFORMATION ACQUISITION DEVICE**

(30) Priority: 07.10.2005 JP 2005294950
(71) Applicant: Olympus Corporation, Tokyo 151-0072 (JP)
(72) Inventor: SUZUSHIMA, Hiroshi, Tokyo 1510072 (JP); FUJIMORI, Noriyuki, Tokyo 1510072 (JP); HOMAN, Masatoshi, Tokyo 1510072 (JP); HONDA, Takemitsu, Tokyo 1510072 (JP); ORIHARA, Tatsuya, Tokyo 1510072 (JP); NAKATSUCHI, Kazutaka, Tokyo 1510072 (JP)
(74) Representative: von Hellfeld, Axel
(86) International application number: PCT/JP2006/320106
(87) International publication number: WO 2007/043473

(57) **Abstract**

It is possible to recognize that the received image data is captured by which imaging element. Respective transmission modules (46a, 46b) assign, to image data captured by a plurality of CCDs (12a, 12b) corresponding respectively to LEDs (11a, 11b), different radio frequencies according to the CCDs (12a, 12b) and simultaneously wirelessly transmit them, for example. A receiving device is provided with a general receiving circuit to receive the radio waves of different radio frequencies, and receives the transmitted image data.

## Description

### TECHNICAL FIELD

The present invention relates to an in-vivo information acquiring apparatus such as a compound-eye type capsule endoscope.

### BACKGROUND ART

In recent years, in the field of endoscopes, a capsule endoscope equipped with an imaging function and a wireless communication function has appeared. The capsule endoscope has a configuration in which during an observation time period from after the capsule endoscope is swallowed by a subject who is a subject from the mouth for observation (examination) until the capsule endoscope naturally comes out of the living body (human body) of the subject, the capsule endoscope moves through internal organs (body cavities), such as esophagus, stomach, small intestine, etc., with their peristalsis and sequentially performs imaging at a predetermined imaging rate by using the imaging function.

During an observation time period in which a capsule endoscope moves through these organs, data on images of the body cavities captured by the capsule endoscope is sequentially transmitted outside a subject, by a wireless communication function such as wireless communication, and accumulated in a memory provided in an external receiver. After observation, a doctor or nurse can make a diagnosis by allowing display means such as a display to display the images of the body cavities based on the data on images accumulated in the memory of the receiver (see Patent Document 1).

Patent Document 1: Japanese Patent Application Laid-Open No. 2003-19111
Patent Document 2: U.S. Patent Application Publication No. 2004/0199061, Specification

### DISCLOSURE OF INVENTION

### PROBLEM TO BE SOLVED BY THE INVENTION

Meanwhile, for this type of capsule endoscope, a monocular type capsule endoscope that images only images of body cavities on the front of the endoscope in its moving direction is common; however, in recent years, a compound-eye type capsule endoscope that captures images on the front and rear of the endoscope in its moving direction for the purpose of extending the field of view upon observation of esophagus, for example, has also been proposed (see Patent Document 2). This compound-eye type capsule endoscope is structured such that a plurality of imaging blocks, each having a pair of illuminating units, such as an LED, for illuminating body cavities and an imaging element, such as a CCD, that captures images of the illuminated body cavities, are provided in the front and rear of a capsule housing and images on the front and rear of the capsule housing in the body cavities in its moving direction are captured.

However, a compound-eye type capsule endoscope disclosed, for example, in Patent Document 2 merely describes imaging of images in both front and rear directions by a plurality of imaging elements and does not mention transmission control for transmitting such image data, and the like, and thus not one with which advantages as the compound-eye type can always be effectively utilized.

The present invention is made in view of the foregoing, and it is an object of the present invention to provide an in-vivo information acquiring apparatus capable of recognizing, at the receiving device side, that the received image data is captured by which imaging element.

### MEANS FOR SOLVING PROBLEM

To solve the problems and achieve the object, an in-vivo information acquiring apparatus according to the present invention includes a plurality of illuminating units that illuminates a body cavity, the illuminating units being disposed within the apparatus; a plurality of imaging units that sequentially captures images of the body cavity illuminated by the illuminating unit, the imaging units pairing up with the illuminating units, respectively; and a transmission unit that transmits information on the images obtained by the imaging units, in a communication mode capable of identifying the imaging units.

The in-vivo information acquiring apparatus as recited in claim 2 according to the invention further includes a frequency assigning unit that assigns different wireless frequencies so that the frequencies correspond to the imaging units, respectively. The transmission unit transmits the information on the images obtained by the imaging units, by using the wireless frequencies assigned to correspond to the imaging units by the frequency assigning unit, as the communication mode.

In the in-vivo information acquiring apparatus as recited in claim 3 according to the invention, the transmission unit includes a plurality of transmission units that are provided so as to correspond to the imaging units, respectively, and the transmission units provided so as to correspond to the imaging units, respectively, transmit the information on the images obtained by the imaging units by using the wireless frequencies assigned by the frequency assigning unit, as the communication mode.

The in-vivo information acquiring apparatus as recited in claim 4 according to the invention further includes a setting unit that sets identification information corresponding to the imaging units, respectively; and an addition unit that adds the identification information corresponding to the imaging units and set by the setting unit, to the information on the images obtained by the imaging units, respectively. The transmission unit sequentially transmits the information on the images added with the identification information by the addition unit, as the communication mode.

The in-vivo information acquiring apparatus as recited in claim 5 according to the invention further includes a setting unit that sets identification information corresponding to the imaging units, respectively; and a frequency changing unit that changes, based on the identification information set by the setting unit, the wireless frequencies assigned by the frequency assigning unit. The transmission unit sequentially transmits the information on the images obtained by the imaging units, respectively, in a time-division manner by using the wireless frequencies changed by the frequency changing unit, as the communication mode.

In the in-vivo information acquiring apparatus as recited in claim 6 according to the invention, the transmission unit includes at least one antenna that transmits a left-handed circularly polarized wave and a right-handed circularly polarized wave. The in-vivo information acquiring apparatus further includes a radio wave assigning unit that assigns one of the left-handed circularly polarized wave and the right-handed circularly polarized wave so that the waves correspond to the imaging units, respectively. The transmission unit transmits the information on the images obtained by the imaging units, respectively, by using one of the left-handed circularly polarized wave and the right-handed circularly polarized wave that are assigned by the radio wave assigning unit, as the communication mode.

The in-vivo information acquiring apparatus as recited in claim 7 according to the invention further includes a setting unit that sets identification information corresponding to the imaging units, respectively; and a radio wave changing unit that changes, based on the identification information set by the setting unit, one of the circularly polarized waves assigned by the radio wave assigning unit to the other one of the circularly polarized waves. The transmission unit sequentially transmits the information on the images obtained by the imaging units, respectively, in a time-division manner by using the circularly polarized wave changed by the radio wave changing unit, as the communication mode.

The in-vivo information acquiring apparatus as recited in claim 8 according to the invention further includes a code assigning unit that assigns different PN codes so that the PN codes correspond to the imaging units, respectively. The transmission unit spreads and modulates the information on the images obtained by the imaging units by using the PN codes assigned by the code assigning unit and transmits the spread and modulated information on the images, as the communication mode.

The in-vivo information acquiring apparatus as recited in claim 9 according to the invention further includes a setting unit that sets identification information corresponding to the imaging units; and a PN code changing unit that changes the PN codes assigned by the code assigning unit, based on the identification information set by the setting unit. The transmission unit spreads and modulates the information on the images obtained by the imaging units, respectively, by using the PN codes changed by the PN code changing unit and sequentially transmits the spread and modulated information on the images in a time-division manner, as the communication mode.

An in-vivo information acquiring apparatus as recited in claim 10 according to the invention includes a receiving unit that receives information on images captured by a plurality of imaging units and transmitted using a plurality of frequencies that are set so as to correspond to the imaging units, respectively; and an identification unit that identifies, based on a frequency of a received signal, an image captured by one of the imaging units.

An in-vivo information acquiring apparatus as recited in claim 11 according to the invention includes a receiving unit that receives information on images captured by a plurality of imaging units and transmitted using one of a left-handed circularly polarized wave and a right-handed circularly polarized wave so that the waves correspond to the imaging units, respectively; and an identification unit that identifies, based on a polarization state of a received signal, an image captured by one of the imaging units.

### EFFECT OF THE INVENTION

The in-vivo information acquiring apparatus according to the present invention transmits the image information acquired by the plurality of imaging units in a communication way that allows identification of the imaging element, thereby providing an advantage that allows the receiving device side to recognize that the received image information is captured by which imaging element.

### BRIEF DESCRIPTION OF DRAWINGS

FIG. 1 is a schematic diagram showing the overall configuration of a wireless in-vivo information acquiring system according to the present invention.
FIG. 2 is a schematic block diagram showing first and second embodiments of an internal circuit configuration of a capsule endoscope shown in FIG. 1.
FIG. 3 is a block diagram showing the first and second embodiments of an internal circuit configuration of a receiving device shown in FIG. 1.
FIG. 4 is a schematic block diagram showing a third embodiment of the internal circuit configuration of the capsule endoscope shown in FIG. 1.
FIG. 5 is a block diagram showing the third embodiment of the internal circuit configuration of the receiving device shown in FIG. 1.
FIG. 6 is a schematic block diagram showing a fourth embodiment of the internal circuit configuration of the capsule endoscope shown in FIG. 1.
FIG. 7 is a schematic block diagram showing a fifth embodiment of the internal circuit configuration of the capsule endoscope shown in FIG. 1.

### EXPLANATIONS OF LETTERS OR NUMERALS

1 Subject
2 Receiving apparatus
2a Radio unit
2b Receiving main body
3 Capsule endoscope
4 Display device
9, 9a, 9b Antenna selector
10, 10a, 10b Receiving circuit
11a, 11b LED
12a, 12b CCD
13 Storage unit
14, 14a, 14b A/D converter
15 Power supply unit
16, 16a, 16b Signal processing unit
17, 17a, 17b Control unit
26a, 26b Control unit
27, 27a, 27b Wireless unit
41a, 41b LED driving circuit
42a, 42b CCD driving circuit
45a, 45b Control circuit
46, 46a, 46b Transmission module
47, 47a, 47b Transmission antenna
48a, 48b ID storage unit
49a, 49b Adding circuit
50, 51, 52 Switch
53 Frequency changer
54 PN sequence changer
A1 to An Receiving antenna

### BEST MODE(S) FOR CARRYING OUT THE INVENTION

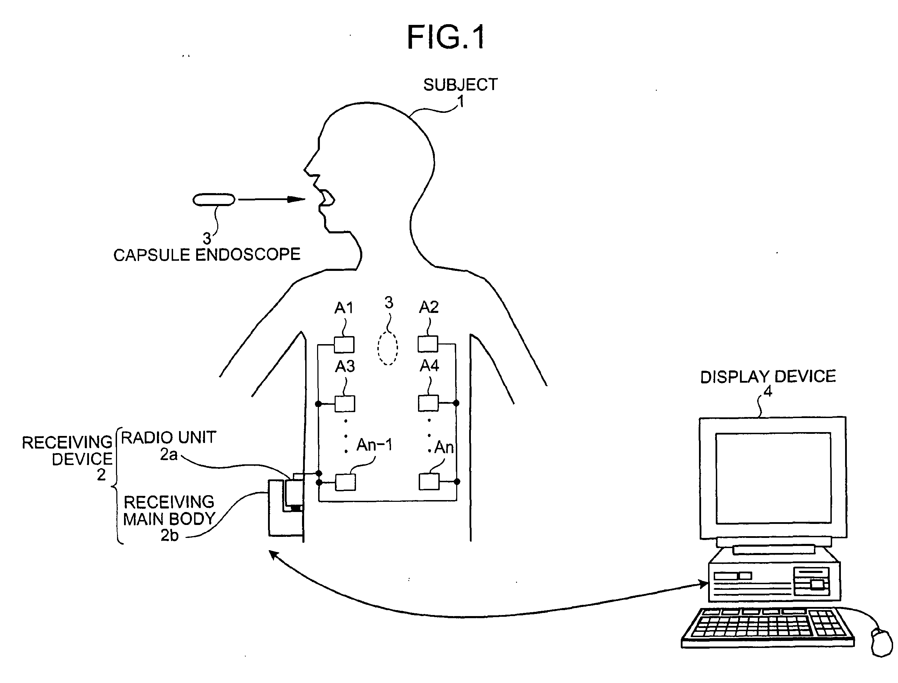

Embodiments of an in-vivo information acquiring apparatus according to the present invention will be described in detail below based on FIGS. 1 to 7. Note that the present invention is not limited to these embodiments and various changes can be made to the embodiments without departing from the sprit and scope of the present invention.

(First embodiment)
FIG. 1 is a schematic diagram showing the overall configuration of a wireless in-vivo information acquiring system according to the present invention. In the in-vivo information acquiring system, a compound-eye type capsule endoscope is used as an exemplary in-vivo information acquiring apparatus. In FIG. 1, the wireless in-vivo information acquiring system includes a capsule endoscope 3 that is introduced into body cavities of a person 1 being tested, captures images of the body cavities, and wirelessly transmits data, such as video signals, to a receiving device 2; and the receiving device 2 that is disposed outside the person 1 being tested and receives data on the images of the body cavities as video signals having been wirelessly transmitted from the capsule endoscope 3. In addition, the in-vivo information acquiring system includes a display device 4 that displays the images of the body cavities based on the video signals received by the receiving device 2. Data passing between the receiving device 2 and the display device 4 is performed by connecting the receiving device 2 to the display device 4 by wire or wirelessly. In the present invention, it is also possible to provide a portable storage medium for performing data passing between the receiving device 2 and the display device 4.

The receiving device 2 includes a radio unit 2a having a plurality of receiving antennas A1 to An attached onto an external body surface of the person 1 being tested; and a receiving main body 2b that processes wireless signals received via the plurality of receiving antennas A1 to An, and the like. These units are removably connected via a connector or the like. Note that the receiving antennas A1 to An may be provided to a jacket that the person 1 being tested can wear, for example, and the person 1 being tested may wear the jacket, whereby the receiving antennas A1 to An may be placed on the person 1 being tested. In this case, the receiving antennas A1 to An may be removable from the jacket.

The display device 4 is to display images of body cavities captured by the capsule endoscope 3, and has a configuration such as a workstation that performs image display based on data obtained by the receiving device 2. Specifically, the display device 4 may be configured to directly display images by means of a CRT display, a liquid crystal display, or the like, or may be configured to output images to another medium, as in a printer.

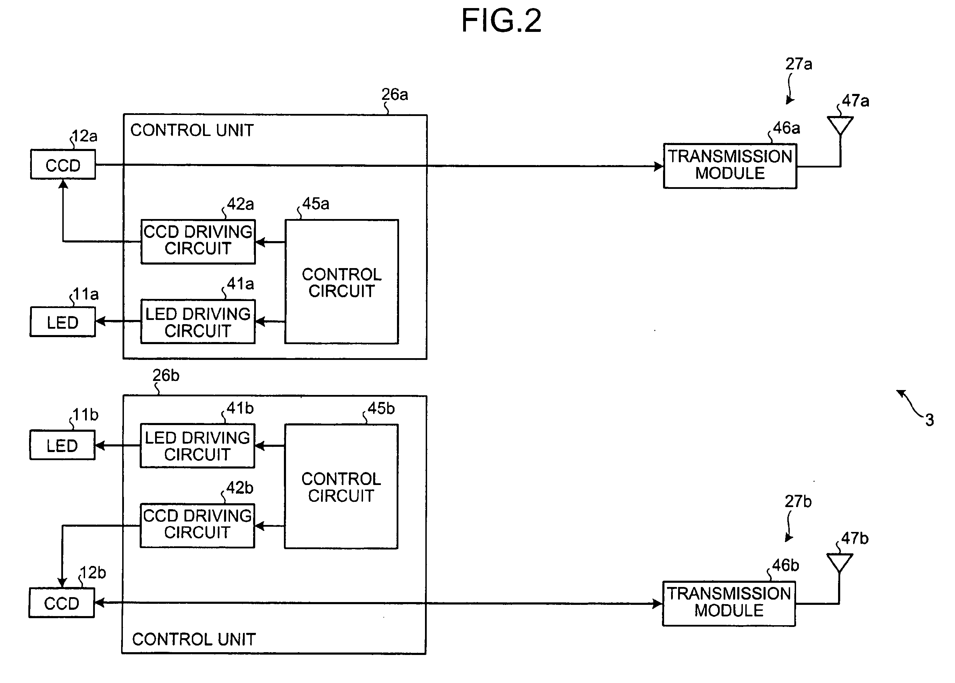

Now, an internal circuit configuration of the capsule endoscope 3 will be described using FIG. 2. FIG. 2 is a schematic block diagram showing the internal circuit configuration of the capsule endoscope 3. In the drawing, a control unit 26a is provided at, for example, the front side of the capsule endoscope 3 (on the right side of the capsule endoscope 3 in a longitudinal direction in FIG. 1) and is to control an LED (light emitting element) 11a which serves as an illuminating unit and a CCD (imaging element) 12a which serves as an imaging unit, the LED 11a pairing up with the CCD 12a. The control unit 26a has an LED driving circuit 41a and a CCD driving circuit 42a provided for the LED 11a and the CCD 12a, respectively. In addition, the control unit 26a includes a control circuit 45a having a timing generator and a sync generator (not shown) that generate various timing signals and synchronization signals. The control circuit 45a controls, for example, the operations and operation timing of the drive circuits 41a and 42a based on timing signals and synchronization signals generated by the timing generator and the sync generator.

A control unit 26b is provided at, for example, the rear side of the capsule endoscope 3 (on the left side of the capsule endoscope 3 in the longitudinal direction in FIG. 1) and is to control an LED 11b which serves as an illuminating unit and a CCD 12b which serves as imaging means, the LED 11b paring up with the CCD 12b. The control unit 26b has an LED driving circuit 41b and a CCD driving circuit 42b provided for the LED 11b and the CCD 12b, respectively. In addition, the control unit 26b includes a control circuit 45b having a timing generator and a sync generator (not shown) that generate various timing signals and synchronization signals. The control circuit 45b controls, for example, the operations and operation timing of the drive circuits 41b and 42b based on timing signals and synchronization signals generated by the timing generator and the sync generator.

A wireless section 27a is composed of a transmission module 46a which serves as a transmission unit provided on an output path for data on images captured by the CCD 12a, for outputting an RF modulation signal; and a transmission antenna 47a. A wireless section 27b is composed of a transmission module 46b which serves as a transmission unit provided on an output path for data on images captured by the CCD 12b, for outputting an RF modulation signal; and a transmission antenna 47b. The transmission modules 46a and 46b have a function as a frequency assigning unit and assign different wireless frequencies so as to correspond to the CCDs 12a and 12b, respectively. In the present embodiment, the wireless frequencies of the transmission modules 46a and 46b are set to fixed frequencies, e.g., 300 MHz and 400 MHz.

The control circuit 45a allows, through the LED driving circuit 41a, the LED 11a to illuminate for a predetermined period of time according to timing signals outputted from both generators and allows the CCD 12a to image that illuminated area. Then, at timing at which the LED 11a goes out, the control circuit 45a allows, through the CCD driving circuit 42a, the CCD 12a to perform an operation of outputting data on images in a frame unit to the transmission module 46. The control circuit 45b allows, through the LED driving circuit 41b, the LED 11b to illuminate for a predetermined period of time according to timing signals outputted from both generators and allows the CCD 12b to image that illuminated area. Then, at timing at which the LED 11b goes out, the control circuit 45b allows, through the CCD driving circuit 42b, the CCD 12b to perform an operation of outputting data on images in a frame unit to the transmission module 46.

By such operations, data on images in a frame unit outputted from the CCD 12a is inputted to the transmission module 46a and is served as a transmission output as RF data with a wireless frequency of 300 MHz, and data on images in a frame unit outputted from the CCD 12b is inputted to the transmission module 46b and is served as a transmission output as RF data with a wireless frequency of 400 MHz.

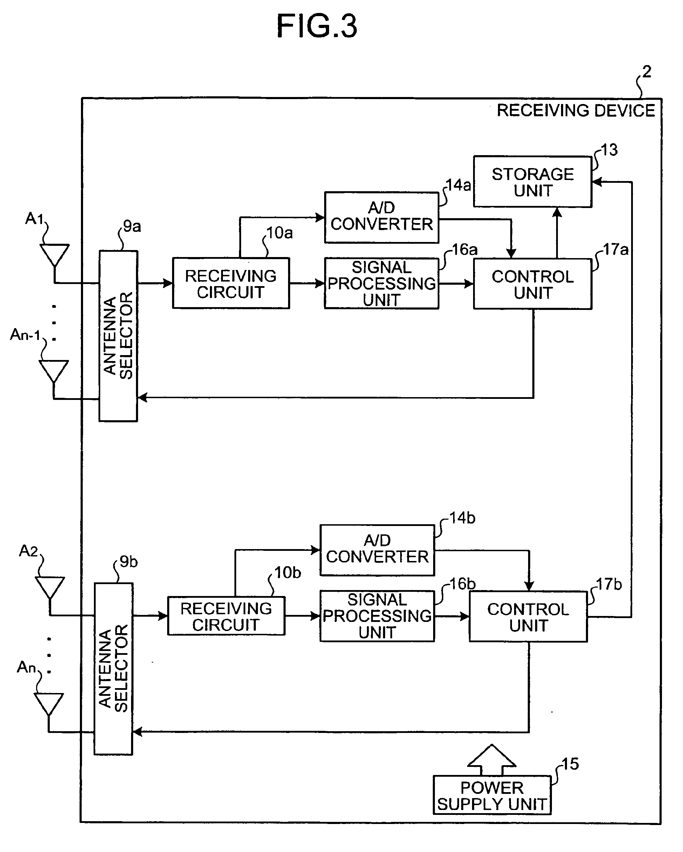

Now, an internal circuit configuration of the receiving device 2 will be described. FIG. 3 is a block diagram showing the internal circuit configuration of the receiving device 2. Note that in the present embodiment a circuit configuration including the radio unit 2a and the receiving main body 2b is shown in FIG. 3 as one block. In the drawing, receiving antennas A1 to An are configured using loop antennas, for example. The receiving antennas A1 to An are used such that they are attached onto predetermined positions of the external body surface of the person 1 being tested. Of the receiving antennas A1 to An, odd-numbered receiving antennas A1 to An-1 receive a wireless signal to be transmitted from the transmission module 46a and even-numbered receiving antennas A2 to An receive a wireless signal to be transmitted from the transmission module 46b.

The receiving device 2 is to perform a process of receiving a wireless signal received via any of the receiving antennas A1 to An-1 and the receiving antennas A2 to An. Specifically, the receiving device 2 according to the present embodiment includes antenna selectors 9a and 9b, each of which selects one antenna suitable for receiving a wireless signal from the plurality of receiving antennas A1 to An-1 and from the plurality of receiving antennas A2 to An; receiving circuits 10a and 10b that perform a process, such as demodulation, on wireless signals received by the receiving antennas selected by the antenna selectors 9a and 9b; and signal processing units 16a and 16b for extracting information on a detected magnetic field, information on images of the inside of the subject, and the like, from the processed wireless signals. The receiving antennas A1 to An-1 and A2 to An, the antenna selectors 9a and 9b, and the receiving circuits 10a and 10b function as receiving units. In addition, the receiving device 2 includes control units 17a and 17b that perform predetermined control on an output of the extracted information, and the like, and function as identifying units; a storage unit 13 that stores the extracted information; A/D converters 14a and 14b that A/D convert analog signals corresponding to the strengths of the received wireless signals outputted from the receiving circuits 10a and 10b; and a power supply unit 15 that supplies a driving power to each component.

The antenna selectors 9a and 9b each have a function of selecting one predetermined receiving antenna based on control of the control units 17a and 17b and outputting wireless signals received by the selected receiving antennas to the receiving circuits 10a and 10b, respectively.

The receiving circuits 10a and 10b each perform predetermined processes, such as amplification and demodulation, on radio wave wireless signals picked up by receiving antennas selected by the antenna selectors 9a and 9b, respectively. Specifically, the receiving circuit 10a has a bandpass filter for capturing a 300 MHz band wireless signal, for example, and receives a wireless signal transmitted from the transmission module 46a. The receiving circuit 10b has a bandpass filter for capturing a 400 MHz band wireless signal, for example, and receives a wireless signal transmitted from the transmission module 46b. In addition, the receiving circuits 10a and 10b each have a function of outputting analog signals corresponding to the strengths of wireless signals to the A/D converters 14, respectively.

The signal processing units 16a and 16b are to extract predetermined information from the signals having been subjected to the predetermined processes by the receiving circuits 10a and 10b. For example, when wireless signals to be received by the receiving device 2 are transmitted from electronic equipment having an imaging function, the signal processing units 16a and 16b extract image data from signals outputted from the receiving circuits 10a and 10b.

The control units 17a and 17b are to perform overall control including an antenna selection operation by the antenna selectors 9a and 9b. Specifically, the control unit 17a transfers information received by the receiving circuit 10a and outputted from the signal processing unit 16a to the storage unit 13 and allows the storage unit 13 to store the information. In addition, the control units 17a and 17b each have a function of determining receiving antennas to be used, based on digital signals (e.g., RSSI (Received Signal Strength Indicators)) that correspond to reception strengths and are outputted from the A/D converters 14a and 14b, respectively, and indicating the antenna selectors 9a and 9b about the determination.

The storage unit 13 is to store the information extracted by the signal processing units 16a and 16b. A specific configuration of the storage unit 13 is such that a memory having at least two storage regions, first and second storage regions, is included to store the information extracted by the signal processing units 16a and 16b in the different first and second storage regions, respectively. The storage unit 13 may have a function of writing information to a portable storage medium.

In the present embodiment, since the transmission module 46a of the capsule endoscope 3 modulates data on images captured by the CCD 12a to a 300 MHz band wireless signal and transmits the wireless signal and the receiving circuit 10a of the receiving device 2 receives a 300 MHz band wireless signal, the control unit 17a can recognize that received data on images is data on images captured by the CCD 12a and can store the data on images in the first storage region of the storage unit 13, for example. Since the transmission module 46b of the capsule endoscope 3 modulates data on images captured by the CCD 12b to a 400 MHz band wireless signal and transmits the wireless signal and the receiving circuit 10b of the receiving device 2 receives a 400 MHz band wireless signal, the control unit 17b can recognize that received data on images is data on images captured by the CCD 12b and can store the data on images in the second storage region of the storage unit 13, for example.

As such, in the present embodiment, since data on images captured by a plurality of CCDs (imaging elements) is wirelessly transmitted by transmission modules assigning different wireless frequencies such that the frequencies correspond to the CCDs, respectively, only by providing general receiving circuits that enable reception of data of these wireless frequency bands to a receiving device side, it becomes possible for the receiving device side to recognize that received image data is data on images captured by either of the imaging elements.

Although in the present embodiment it is configured such that the CCDs 12a and 12b image images on the front and rear of the capsule endoscope 3 in its moving direction, the present invention is not limited thereto; the CCDs 12a and 12b may be provided in the capsule endoscope 3 so as to image images on the left and right in the moving direction, for example, or the CCDs 12a and 12b may be provided in the capsule endoscope 3 such that an optical axis direction (imaging direction) of the CCDs 12a and 12b is not parallel but is a diagonal direction to the center of an axle of the capsule endoscope 3 itself. The number of CCDs, imaging elements, is not limited to two and three or more CCDs may be provided.

(Second embodiment)
Now, a second embodiment of the capsule endoscope 3 according to the present invention will be described. Note that since an internal circuit configuration of the capsule endoscope 3 in the present embodiment is the same as that of FIG. 2, only differences made from a first embodiment will be described with reference to FIG. 2. In addition, since an internal circuit configuration of a receiving device 2 is the same as that of FIG. 3, the description thereof will be made with reference to FIG. 3. In FIG. 2, transmission antennas 47a and 47b are composed of, for example, batch antennas for transmitting circularly polarized waves. Specifically, the transmission antennas 47a and 47b each have a function of a radio wave assigning unit. The transmission antenna 47a is composed of a batch antenna that performs assignment so as to transmit a left-handed circularly polarized wave corresponding to a CCD 12a and the transmission antenna 47b is composed of a batch antenna that performs assignment so as to transmit a right-handed circularly polarized wave corresponding to a CCD 12b.

In FIG. 3, receiving antennas A1 to An-1 of the receiving device 2 each are composed of, for example, a batch antenna that receives a left-handed circularly polarized wave transmitted from the transmission antenna 47a and receiving antennas A2 to An each are composed of, for example, a batch antenna that receives a right-handed circularly polarized wave transmitted from the transmission antenna 47b.

Receiving circuits 10a and 10b perform predetermined processes, such as amplification and demodulation, on circularly polarized wave wireless signals picked up by receiving antennas selected by antenna selectors 9a and 9b, and function, together with the receiving antennas A1 to An-1 and A2 to An and the antenna selectors 9a and 9b, as receiving units. That is, the receiving circuit 10a receives a left-handed circularly polarized wave transmitted from the transmission antenna 47a and picked up, as with the first embodiment, by a receiving antenna selected from the receiving antennas A1 to An-1. The receiving circuit 10b receives a right-handed circularly polarized wave transmitted from the transmission antenna 47b and picked up, as with the first embodiment, by a receiving antenna selected from the receiving antennas A2 to An.

In the present embodiment, since a transmission module 46a of the capsule endoscope 3 modulates data on images captured by the CCD 12a to a wireless RF signal, a wireless left-handed circularly polarized wave signal is transmitted from the transmission antenna 47a, and the receiving circuit 10a of the receiving device 2 receives a wireless left-handed circularly polarized wave signal from any of the receiving antennas A1 to An-1 and demodulates the signal to data on images, a control unit 17a functioning as an identifying unit can recognize that received data on images is data on images captured by the CCD 12a and can store the data on images in a first storage region of a storage unit 13, for example. Since a transmission module 46b of the capsule endoscope 3 modulates data on images captured by the CCD 12b to a wireless RF signal, a wireless right-handed circularly polarized wave signal is transmitted from the transmission antenna 47b, and the receiving circuit 10b of the receiving device 2 receives a wireless right-handed circularly polarized wave signal from any of the receiving antennas A2 to An and demodulates the signal to data on images, a control unit 17b functioning as an identifying unit can recognize that received data on images is data on images captured by the CCD 12b and can store the data on images in a second storage region of the storage unit 13, for example.

As such, in the present embodiment, since data on images captured by a plurality of CCDs (imaging elements) is transmitted by transmission antennas corresponding to the CCDs, respectively, using a wireless left-handed circularly polarized wave signal or a wireless right-handed circularly polarized wave signal, only by providing general receiving circuits that enable reception of data of these left-handed circularly polarized wave and right-handed circularly polarized wave to a receiving device side, it becomes possible for the receiving device side to recognize that received image data is data on images captured by either of the imaging elements.

(Third embodiment)

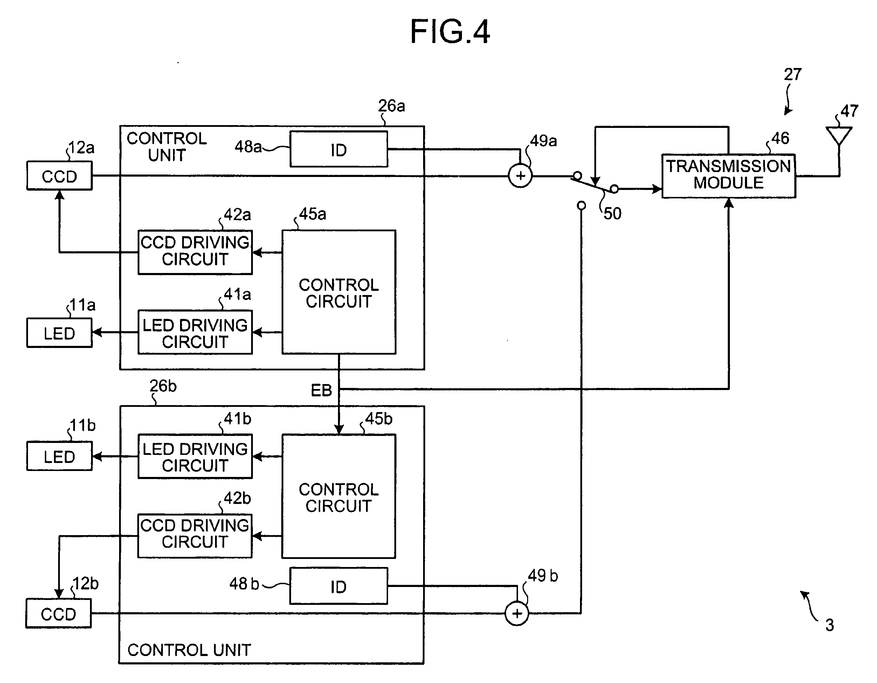

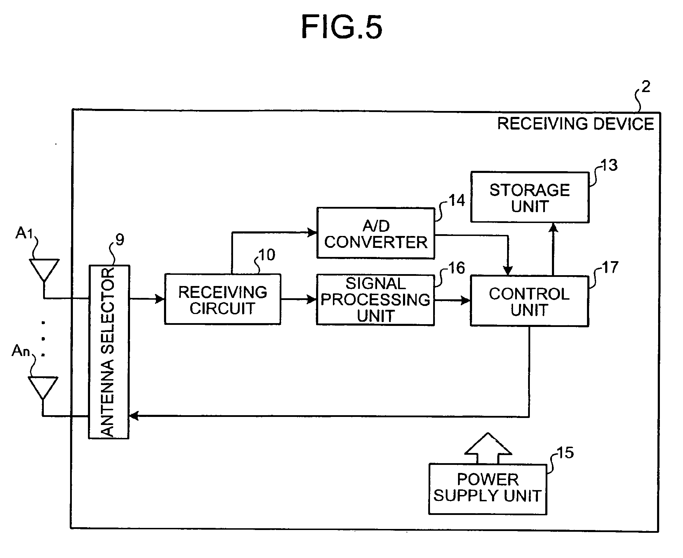

FIG. 4 is a schematic block diagram showing a third embodiment of an internal circuit configuration of a capsule endoscope 3. FIG. 5 is a block diagram showing the third embodiment of an internal circuit configuration of a receiving device 2. The present embodiment is different from the first embodiment in that ID storage units 48a and 48b as setting units for storing an ID (identification information) for identifying data on images captured by either of CCDs 12a and 12b, are provided in control units 26a and 26b so as to correspond to the CCDs 12a and 12b, respectively, and adding circuits 49a and 49b as addition units for adding the ID to data on images captured by the CCDs 12a and 12b are provided. In the present embodiment, the data on images added with the ID is alternately transmitted in a time-division manner from a transmission module 46.

Control circuits 45a and 45b have a master/slave relationship in which the control circuit 45a is a master and the control circuit 45b is a slave. The control circuit 45b performs, according to an enable signal EB from the control circuit 45a, a control operation in accordance with the control circuit 45a such that it operates only during a period of time in which the enable signal EB is a high level, for example.

Here, the control circuits 45a and 45b allow the CCDs 12a and 12b to alternately and sequentially drive and control timing such that illumination timing of LEDs 11a and 11b differs from output timing of the CCDs 12a and 12b. That is, the control circuit 45a first allows the LED 11a which pairs up with the CCD 12a to illuminate for a predetermined period of time. Then, after a subsequent operation of outputting data on images on the front side from the CCD 12a is completed, the control circuit 45b allows the LED 11b which pairs up with the CCD 12b to illuminate for a predetermined period of time. Then, a subsequent operation of outputting data on images on the rear side from the CCD 12b is performed. Thereafter, such operation control is repeated.

More specifically, the control circuit 45a allows, through an LED driving circuit 41a, the LED 11a to illuminate for the predetermined period of time according to timing signals to be outputted from both generators and allows the CCD 12a to image that illuminated area. Then, at timing at which the LED 11a goes out, the control circuit 45a allows, through the CCD driving circuit 42a, the CCD 12a to perform an operation of outputting data on images in a frame unit to the transmission module 46. When this output operation is completed, the control circuit 45a outputs an enable signal EB (high level) to the control circuit 45b and the transmission module 46 and switches to control by the control circuit 45b.

The control circuit 45b performs a control operation in response to an input of an enable signal EB (high level), allows, through an LED driving circuit 41b, the LED 11b to illuminate for the predetermined period of time according to timing signals to be outputted from both generators, and allows the CCD 12b to image that illuminated area. Then, at timing at which the LED 11b goes out, the control circuit 45b allows, through the CCD driving circuit 42b, the CCD 12b to perform an operation of outputting data on images in a frame unit to the transmission module 46. At timing at which this output operation is completed, the control circuit 45a switches the enable signal EB to a low level and thereby switches to control by the control circuit 45a. Thereafter, such operation control is repeated. It is also possible that, in a circuit configuration, by the control circuit 45b outputting an end signal to the control circuit 45a upon completion of an output, the control circuit 45a may switch the enable signal EB to a low level.

By such an operation, data on images in a frame unit to be alternately and sequentially outputted from the CCDs 12a and 12b is outputted to the transmission module 46, and the transmission module 46 detects output timing of the data on images by an enable signal EB to be inputted thereto, switches a switch 50 to the side of the CCD 12a or the side of the CCD 12b in synchronization with the output timing, and captures data on images. Then, the transmission module 46 modulates data on images to be inputted thereto to a wireless RF signal and sequentially transmits the wireless RF signal in a time-division manner.

In FIG. 5, the receiving device 2 includes an antenna selector 9 that selects an antenna suitable for receiving a wireless signal from receiving antennas A1 to An; a receiving circuit 10 that performs a process, such as demodulation, on the received wireless signal; a signal processing unit 16 for extracting data on images and the like from the wireless signal; a control unit 17 that performs predetermined control; a storage unit 13 that stores the extracted information in a first or second storage region; an A/D converter 14 that A/D converts an analog signal corresponding to the strength of the received wireless signal; and a power supply unit 15.

In this configuration, a radio wave wireless signal picked up by a receiving antenna selected by the antenna selector 9 is subjected to predetermined processes, such as amplification and demodulation, by the receiving circuit 10. The signal processing unit 16 extracts data on images in a frame unit and an ID from the signal outputted from the receiving circuit 10. The control unit 17 can identify, based on the extracted ID, either of the CCDs 12a and 12b having obtained the data on images and can further allow the image data to be stored in the first or second storage region.

As such, in the present embodiment, since data on images captured by a plurality of CCDs (imaging elements) is added with IDs corresponding to the CCDs and the data on images from the CCDs, respectively, is sequentially transmitted in a time-division manner, it becomes possible for a receiving device side to recognize based on the ID that received image data is data on images captured by either of the imaging elements.

In the present embodiment, by setting a clock speed for allowing the transmission module 46 to operate to a speed twice, for example, as fast as a clock speed for allowing the CCDs 12a and 12b to operate, it becomes possible to transmit data on images captured by the CCDs 12a and 12b to the receiving device 2 in real time and in a time-division manner.

(Fourth embodiment)

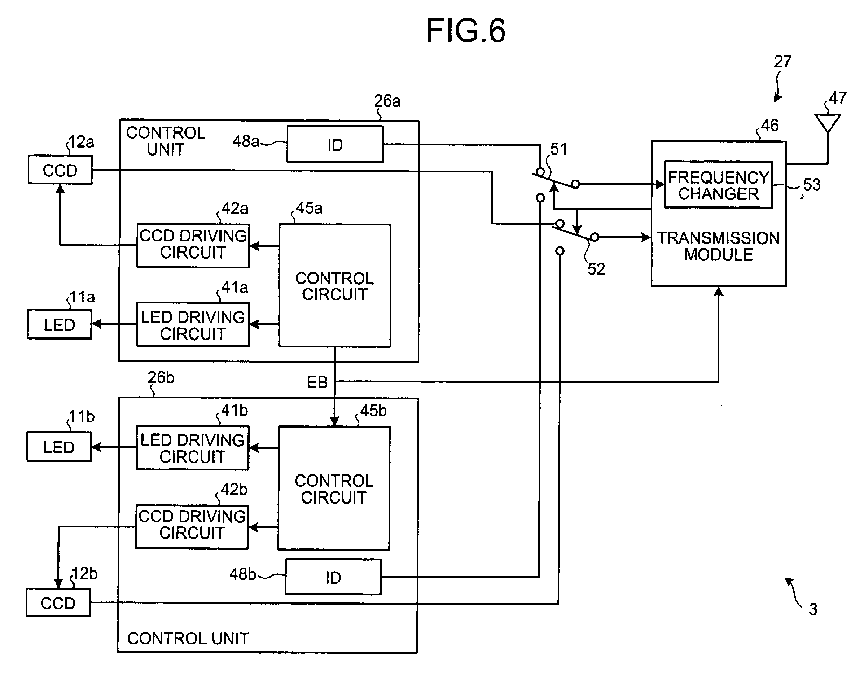

FIG. 6 is a schematic block diagram showing a fourth embodiment of an internal circuit configuration of a capsule endoscope 3. In the present embodiment, as with the first embodiment, different wireless frequencies are assigned so as to correspond to CCDs 12a and 12b, respectively, and wireless transmission is performed by a transmission module; however, the present embodiment is different from the first embodiment in that ID storage units 48a and 48b as setting units for storing an ID for identifying data on images captured by either of the CCDs 12a and 12b, are provided in control units 26a and 26b so as to correspond to the CCDs 12a and 12b, respectively (as with the third embodiment), a frequency changer 53 as a frequency assigning and frequency changing unit for assigning and changing wireless frequencies corresponding to the CCDs 12a and 12b, respectively, based on the IDs in the ID storage units 48a and 48b is provided in the transmission module 46, and the single transmission module 46 sequentially transmits data on images from the CCDs 12a and 12b in a time-division manner.

Control circuits 45a and 45b have a master/slave relationship in which the control circuit 45a is a master and the control circuit 45b is a slave, and perform the same control operation as that in the third embodiment. In the present embodiment, data on images in a frame unit to be alternately and sequentially outputted from the CCDs 12a and 12b and IDs corresponding to the CCDs 12a and 12b, respectively, are outputted to the transmission module 46, and the transmission module 46 detects output timing of the data on images by an enable signal EB to be inputted thereto, switches switches 51 and 52 to the side of the CCD 12a or the side of the CCD 12b in synchronization with the output timing, and captures data on images and an ID.

In the frequency changer 53 of the transmission module 46, IDs corresponding to the CCDs 12a and 12b, respectively, and wireless frequencies are set so as to be associated with each other and the frequency changer 53 changes, based on an ID to be inputted from either of the control units 26a and 26b, a corresponding wireless frequency. Specifically, when an ID corresponding to the CCD 12a is inputted, the frequency changer 53 changes, based on the ID, the wireless frequency of the transmission module 46 to 300 MHz, for example, and when an ID corresponding to the CCD 12b is inputted, the frequency changer 53 changes, based on the ID, the wireless frequency of the transmission module 46 to 400 MHz, for example. Then, the transmission module 46 modulates data on images to be inputted from the CCD 12a to RF data with a wireless frequency of 300 MHz and modulates data on images to be inputted from the CCD 12b to RF data with a wireless frequency of 400 MHz and then sequentially transmits those RF data in a time-division manner.

A receiving device 2 is of the same configuration as that in FIG. 3, for example. The receiving device 2 receives a 300 MHz band wireless signal by a receiving circuit 10a, receives a 400 MHz band wireless signal by a receiving circuit 10b, and performs a process, such as demodulation, on the received wireless signals. Control units 17a and 17b transfer information received by the receiving circuits 10a and 10b and outputted from the signal processing units 16a and 16b to a storage unit 13 and allows the storage unit 13 to store the information.

As such, in the present embodiment, since wireless frequencies assigned to a transmission module are changed based on IDs corresponding to CCDs, respectively, and data on images from the CCDs, respectively, is sequentially transmitted in a time-division manner by using the changed wireless frequencies, only by providing general receiving circuits that enable reception of data of these wireless frequency bands to a receiving device side, it becomes possible for the receiving device side to recognize that received image data is data on images captured by either of imaging elements.

(Fifth embodiment)

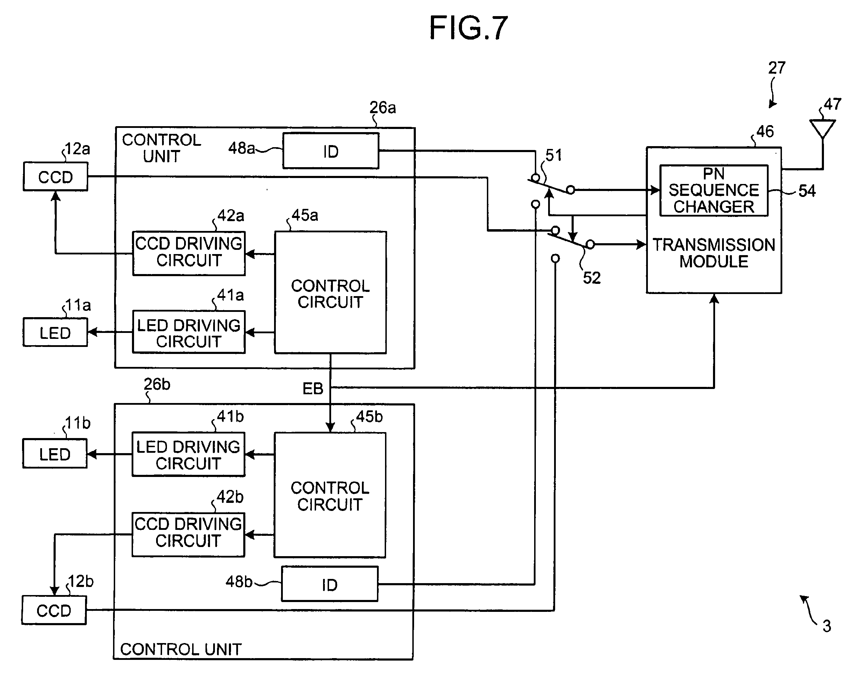

FIG. 7 is a schematic block diagram showing a fifth embodiment of an internal circuit configuration of a capsule endoscope 3. The present embodiment is different from the fourth embodiment in that a communication mode performs wireless transmission of data on images by a spread spectrum using a PN code and a PN sequence changer 54 as a code assigning unit for assigning different PN codes (pseudo noises) corresponding to CCDs 12a and 12b, respectively, and as a PN code changing unit for changing the assigned PN codes based on IDs from ID storage units 48a and 48b as setting units is provided in a transmission module 46 instead of the frequency changer 53.

In the PN sequence changer 54, pieces of information on IDs corresponding to the CCDs 12a and 12b, respectively, and pieces of information on PN codes are set so as to be associated with each other and the PN sequence changer 54 changes, based on an ID to be inputted from either of control units 26a and 26b, a corresponding PN code. Specifically, when an ID identifying the CCD 12a is inputted, the PN sequence changer 54 changes the PN code to a first PN code that corresponds to the ID, and when an ID identifying the CCD 12b is inputted, the PN sequence changer 54 changes the PN code to a second PN code that corresponds to the ID. The first PN code and the second PN code are composed of different code sequences.

The transmission module 46 performs a primary modulation such as PSK on data on images from the CCDs 12a and 12b, respectively, and thereafter performs a secondary modulation (spread modulation) on the data on images using a PN code outputted from the PN sequence changer 54. Specifically, the transmission module 46 performs a primary modulation on data on images to be inputted from the CCD 12a and thereafter performs a spread modulation on the data on images by using the first PN code. Then, the transmission module 46 performs likewise a primary modulation on data on images to be inputted from the CCD 12b and thereafter performs a spread modulation on the data on images by using the second PN code, and then sequentially transmits those data on images in a time-division manner.

A receiving device 2 is of the same configuration as that in FIG. 3. A receiving circuit 10a inversely spreads a wireless signal using the first PN code and receives the wireless signal, and a receiving circuit 10b inversely spreads a wireless signal using the second PN code and receives the wireless signal. Control units 17a and 17b transfer information received by the receiving circuits 10a and 10b and outputted from signal processing units 16a and 16b to a storage unit 13 and allows the storage unit 13 to store the information.

As such, in the present embodiment, since PN codes assigned to a transmission module are changed based on IDs corresponding to CCDs, respectively, data on images from the CCDs, respectively, is spread and modulated by using the changed PN codes, and the image data is sequentially transmitted in a time-division manner, only by providing general receiving circuits that enable reception of these spread data to a receiving device side, it becomes possible for the receiving device side to recognize that received image data is data on images captured by either of imaging elements.

In addition, in the present embodiment, by using spread spectrum communication, it becomes possible to transmit a signal less susceptible to outside noise. Thus, data on images with little noise can be transmitted to a receiving device and accordingly it becomes possible to make a diagnosis by a doctor or the like more easily and reliably.

(Modification)
Although the fourth and fifth embodiments describe the case of changing a wireless frequency and a PN code, the present invention is not limited thereto; for example, the invention can also be applied to the case of changing a circularly polarized wave described in the second embodiment. In this variant, a transmission antenna 47a for transmitting a left-handed circularly polarized wave and a transmission antenna 47b for transmitting a right-handed circularly polarized wave are connected, through a switching switch, to a transmission module 46 so as to be switchable. Then, a radio wave changer as a radio wave changing unit for changing from one circularly polarized wave to the other circularly polarized wave is provided in the transmission module 46. The radio wave changer switches, based on IDs to be inputted that correspond to CCD 12a and 12b, respectively, a connection from one transmission antenna (e.g., the transmission antenna 47a) that is assigned (connected) to the transmission module 46 to the other transmission antenna (e.g., the transmission antenna 47b) and thereby changes a radio wave to be transmitted from one circularly polarized wave (e.g., a left-handed circularly polarized wave) to the other circularly polarized wave (e.g., a right-handed circularly polarized wave). A wireless section 27 can then sequentially transmit data on images from the CCDs 12a and 12b, respectively, in a time-division manner by using the assigned circularly polarized waves.

As such, in the variant, since circularly polarized waves assigned to a transmission module are changed based on IDs corresponding to CCDs, respectively, and data on images from the CCDs, respectively, is sequentially transmitted in a time-division manner by using the changed circularly polarized waves, only by providing general receiving circuits that enable reception of these left-handed circularly polarized wave and right-handed circularly polarized wave to a receiving device side, it becomes possible for the receiving device side to recognize that received data on images is data on images captured by either of imaging elements.

### INDUSTRIAL APPLICABILITY

As described above, the in-vivo information acquiring apparatus is suitable for a compound-eye type capsule microscope and the like, and is applicable to capturing the front, rear, and both sides images by the plurality of imaging units.

## Claims

1. An in-vivo information acquiring apparatus, comprising:
a plurality of illuminating units that illuminates a body cavity, the illuminating units being disposed within the apparatus;
a plurality of imaging units that sequentially captures images of the body cavity illuminated by the illuminating unit, the imaging units pairing up with the illuminating units, respectively; and
a transmission unit that transmits information on the images obtained by the imaging units, in a communication mode capable of identifying the imaging units.

2. The in-vivo information acquiring apparatus according to claim 1, further comprising a frequency assigning unit that assigns different wireless frequencies so that the frequencies correspond to the imaging units, respectively, wherein
the transmission unit transmits the information on the images obtained by the imaging units, by using the wireless frequencies assigned to correspond to the imaging units by the frequency assigning unit, as the communication mode.

3. The in-vivo information acquiring apparatus according to claim 2, wherein the transmission unit includes a plurality of transmission units that are provided so as to correspond to the imaging units, respectively, and the transmission units provided so as to correspond to the imaging units, respectively, transmit the information on the images obtained by the imaging units by using the wireless frequencies assigned by the frequency assigning unit, as the communication mode.

4. The in-vivo information acquiring apparatus according to claim 2, further comprising:
a setting unit that sets identification information corresponding to the imaging units, respectively; and
an addition unit that adds the identification information corresponding to the imaging units and set by the setting unit, to the information on the images obtained by the imaging units, respectively, wherein
the transmission unit sequentially transmits the information on the images added with the identification information by the addition unit, as the communication mode.

5. The in-vivo information acquiring apparatus according to claim 2, further comprising:
a setting unit that sets identification information corresponding to the imaging units, respectively; and
a frequency changing unit that changes, based on the identification information set by the setting unit, the wireless frequencies assigned by the frequency assigning unit, wherein
the transmission unit sequentially transmits the information on the images obtained by the imaging units, respectively, in a time-division manner by using the wireless frequencies changed by the frequency changing unit, as the communication mode.

6. The in-vivo information acquiring apparatus according to claim 1, wherein
the transmission unit includes at least one antenna that transmits a left-handed circularly polarized wave and a right-handed circularly polarized wave,
the in-vivo information acquiring apparatus further comprises a radio wave assigning unit that assigns one of the left-handed circularly polarized wave and the right-handed circularly polarized wave so that the waves correspond to the imaging units, respectively, and
the transmission unit transmits the information on the images obtained by the imaging units, respectively, by using one of the left-handed circularly polarized wave and the right-handed circularly polarized wave that are assigned by the radio wave assigning unit, as the communication mode.

7. The in-vivo information acquiring apparatus according to claim 6, further comprising:
a setting unit that sets identification information corresponding to the imaging units, respectively; and
a radio wave changing unit that changes, based on the identification information set by the setting unit, one of the circularly polarized waves assigned by the radio wave assigning unit to the other one of the circularly polarized waves, wherein
the transmission unit sequentially transmits the information on the images obtained by the imaging units, respectively, in a time-division manner by using the circularly polarized wave changed by the radio wave changing unit, as the communication mode.

8. The in-vivo information acquiring apparatus according to claim 1, further comprising a code assigning unit that assigns different PN codes so that the PN codes correspond to the imaging units, respectively, wherein
the transmission unit spreads and modulates the information on the images obtained by the imaging units by using the PN codes assigned by the code assigning unit and transmits the spread and modulated information on the images, as the communication mode.

9. The in-vivo information acquiring apparatus according to claim 8, further comprising:
a setting unit that sets identification information corresponding to the imaging units; and
a PN code changing unit that changes the PN codes assigned by the code assigning unit, based on the identification information set by the setting unit, wherein
the transmission unit spreads and modulates the information on the images obtained by the imaging units, respectively, by using the PN codes changed by the PN code changing unit and sequentially transmits the spread and modulated information on the images in a time-division manner, as the communication mode.

10. An in-vivo information acquiring apparatus comprising:
a receiving unit that receives information on images captured by a plurality of imaging units and transmitted using a plurality of frequencies that are set so as to correspond to the imaging units, respectively; and
an identification unit that identifies, based on a frequency of a received signal, an image captured by one of the imaging units.

11. An in-vivo information acquiring apparatus, comprising:
a receiving unit that receives information on images captured by a plurality of imaging units and transmitted using one of a left-handed circularly polarized wave and a right-handed circularly polarized wave so that the waves correspond to the imaging units, respectively; and
an identification unit that identifies, based on a polarization state of a received signal, an image captured by one of the imaging units.
